(19)

Europäisches
Patentamt
European
Patent Office
Office européen
des brevets

(11) **EP 2 407 179 A1**

(12) **EUROPEAN PATENT APPLICATION**
published in accordance with Art. 153(4) EPC

(43) Date of publication:
**18.01.2012 Bulletin 2012/03**

(21) Application number: **10750600.8**

(22) Date of filing: **11.03.2010**

(51) Int Cl.:
*A61K 45/00* (2006.01)    *A61K 9/06* (2006.01)
*A61K 9/08* (2006.01)    *A61K 9/12* (2006.01)
*A61K 9/70* (2006.01)    *A61K 31/196* (2006.01)
*A61K 47/06* (2006.01)    *A61K 47/08* (2006.01)
*A61K 47/10* (2006.01)    *A61K 47/18* (2006.01)
*A61K 47/34* (2006.01)    *A61P 29/00* (2006.01)

(86) International application number:
**PCT/JP2010/001761**

(87) International publication number:
**WO 2010/103844 (16.09.2010 Gazette 2010/37)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **11.03.2009 JP 2009057973**

(71) Applicant: **Kowa Company, Ltd.**
**Nagoya-shi, Aichi-ken 460-8625 (JP)**

(72) Inventors:
• **MIURA, Seiji**
**Fuji-shi**
**Shizuoka 417-8650 (JP)**

• **AWAMURA, Tsutomu**
**Imizu-shi**
**Toyama 939-0351 (JP)**
• **YAMAZAKI, Yuhiro**
**Imizu-shi**
**Toyama 939-0351 (JP)**
• **FUJII, Hironari**
**Imizu-shi**
**Toyama 939-0351 (JP)**

(74) Representative: **Blodig, Wolfgang**
**Wächtershäuser & Hartz**
**Patentanwaltspartnerschaft**
**Weinstraße 8**
**80333 München (DE)**

(54) **EXTERNAL PREPARATION CONTAINING ANALGESIC/ANTI-INFLAMMATORY AGENT**

(57)    An external preparation containing the following components (A) and (B):
(A) a non-steroidal analgesic/anti-inflammatory agent, and
(B) an organic amine. The external preparation of the present invention has improved skin permeation and excellent stability of a non-steroidal analgesic/anti-inflammatory agent in the external preparation. The external preparation of the present invention also has excellent appearance.

EP 2 407 179 A1

**Description**

[Technical Field]

The present invention relates to an external preparation containing a non-steroidal analgesic/anti-inflammatory agent.

[Background Art]

Amfenac or a salt thereof, a phenyl acetate type non-steroidal anti-inflammatory analgesic agent, is indicated for relieving inflammation and pain associated with chronic rheumatoid arthritis, osteoarthritis, low back pain, scapulohumeral periarthritis, cervico-omo-brachial syndrome, and temporomandibular arthrosis as well as following surgery, injury, tooth extraction, and the like, and a capsule containing 50 mg of amfenac sodium per capsule is used. However, because of the short blood half-life of amfenac or a salt thereof, four times-daily administration has been necessary for oral administration. Also, because amfenac or a salt thereof inhibits biosynthesis of prostaglandin, there is a possibility of digestive tract mucosal injury being caused as a side effect (Non-Patent Document 1).

In order to solve such a problem, it has been demanded that amfenac or a salt thereof be provided as an external preparation. As an external preparation containing amfenac sodium, for example, a patch for external application in which an acrylic adhesive layer is provided on a support (refer to Patent Document 1) and an anti-inflammatory analgesic patch in which a pressure sensitive adhesive material layer containing an organic acid more strongly acidic than amfenac in the free state is laminated onto a flexible support (refer to Patent Document 2) are known. However, amfenac sodium is a compound exhibiting a deep yellow color and there is a tendency that the color tone becomes stronger in a manner dependent on the concentration. There is concern that an external preparation with strong color tone may cause staining, etc., if it adheres to clothes upon application. Thus, it is required that amfenac sodium be prepared into a well-absorbable pharmaceutical preparation in a range of low concentration, within which it appears light yellow in color. However, either of the external preparations described in Patent Documents 1 and 2 contains such a high concentration of amfenac sodium as 5% by weight or more, leaving the aforementioned problem unsolved.

Further, amfenac or a salt thereof is extremely unstable to an acidic substance, and there is concern that the stability of the external preparation described in Patent Document 2 may be decreased by incorporating a strongly acidic organic acid.

Meanwhile, various technologies for improving the percutaneous absorbability of a non-steroidal analgesic/anti-inflammatory agent have also been proposed. For example, a ketoprofen ointment prepared with an oily base composed of fatty acid ester, waxes, surfactants, and hydrocarbons (refer to Patent Document 3), a ketoprofen ointment prepared with an emulsion base composed of higher alcohol, hydrocarbons, water, and emulsifiers (see Patent Document 4), an indometacin-containing liquid preparation prepared by blending a specific polyoxyethylene-based nonionic surfactant in a lower alcohol-water-based base containing vitamin Es and medium chain fatty acid ester (see Patent Document 5), a non-steroidal anti-inflammatory analgesic agent-containing patch for external application containing alkyl pyrrolidone, hydrophilic polyether, hydrophilic nonionic surfactants, water-soluble polymers having a carboxyl group, water-soluble vinyl polymers, water-insoluble multivalent metal salts, polyhydric alcohol, organic hydroxy acid, and water (see Patent Document 6), a piroxicam-containing transdermal system containing, as an adhesive, a copolymer composed of N-vinyl-2-pyrrolidone and (meth)acrylic acid ester, as a drug solubilizing aid, polyvinyl pyrrolidone, and as a penetration enhancer, polyoxyethylene alkyl ether and/or fatty acid alkylolamide (see Patent Document 7), a ketoprofen liquid preparation for external application containing polyoxyethylenepolyoxypropylene alkyl ether, hydroxyalkyl cellulose, isopropyl adipate, and/or isopropyl myristate, a mixture of water and ethanol, and the like (see Patent Document 8), and an external preparation containing oleic acid or oleyl alcohol and a non-steroidal anti-inflammatory analgesic agent in an aqueous alcohol solvent (see Patent Document 9) are known.

However, none of these documents specifically describes or suggests improvement of the percutaneous absorbability of amfenac or a salt thereof, and improvement of the stability of amfenac or a salt thereof contained in the external preparation by use of an organic amine in combination.

[Prior Art Document]

[Patent Document]

[Patent Document 1] JP-A-61-126020
[Patent Document 2] JP-A-62-126119

[Patent Document 3] JP-A-58-39616
[Patent Document 4] JP-A-58-103311
[Patent Document 5] JP-A-6-9394
[Patent Document 6] JP-A-2002-20274
[Patent Document 7] JP-A-3-251534
[Patent Document 8] JP-A-1-143831
[Patent Document 9] JP-A-2000-143540

[Non-Patent Document]

[Non-Patent Document 1] "Drugs in Japan, Ethical drugs, 2006", Jiho, Inc., page 221

[Summary of Invention]

An object of the present invention is to provide an external preparation containing a non-steroidal analgesic/ anti-inflammatory agent (particularly, amfenac or a salt thereof) which has excellent skin permeation and stability.

The present inventors conducted a study on an external preparation containing a non-steroidal analgesic/anti-inflammatory agent. As a result, they have found that a pharmaceutical preparation in which the stability and the transdermal absorbability of a non-steroidal analgesic/anti-inflammatory agent are markedly improved and which also has excellent appearance can be obtained by mixing an organic amine.

The present invention provides an external preparation containing the following components (A) and (B) :

(A) a non-steroidal analgesic/anti-inflammatory agent,
(B) an organic amine.

The external preparation of the present invention has improved skin permeation and excellent stability of a non-steroidal analgesic/anti-inflammatory agent in the external preparation. The external preparation of the present invention also has excellent appearance.

[Modes for Carrying out the Invention]

[Component (A): Non-steroidal analgesic/anti-inflammatory agent]

Although no particular limitation is imposed on the non-steroidal analgesic/anti-inflammatory agent of Component (A) used in the present invention, examples thereof include actarit, acemetacin, ampiroxicam, amfenac, ibuprofen, indometacin, etodolac, ketoprofen, zaltoprofen, diclofenac, sulindac, celecoxib, tiaprofenic acid, tenoxicam, naproxen, piroxicam, felbinac, pranoprofen, flurbiprofen, mefenamic acid, medicoxib, meloxicam, mofezolac, refecoxib, loxoprofen, lobenzarit, lornoxicam, and a salt of these substances. Among them, amfenac or a salt thereof is preferable. More specific examples include actarit, acemetacin, ampiroxicam, amfenac sodium, ibuprofen, indometacin, indometacin farnesil, etodolac, ketoprofen, zaltoprofen, diclofenac sodium, sulindac, celecoxib, tiaprofenic acid, tenoxicam, naproxen, piroxicam, felbinac, pranoprofen, flurbiprofen, flurbiprofen axetil, mefenamic acid, medicoxib, meloxicam, mofezolac, refecoxib, loxoprofen sodium hydrate, lobenzarit disodium, and lornoxicam, and amfenac sodium (chemical name: sodium (2-amino-3-benzoylphenyl)acetate monohydrate) is particularly preferable.

In the external preparation of the present invention, the non-steroidal analgesic/anti-inflammatory agent of Component (A) can be used singly or in combination of two or more. Although no particular limitation is imposed on the content thereof, it is preferably 0.001 to 20% by mass, more preferably 0.01 to 10% by mass, and particularly preferably 0.05 to 5% by mass. It is to be noted that the content of each component of the external preparation of the present invention refers to, unless otherwise specifically noted, the mass ratio of each component to the total mass of "a part containing Components (A) and (B)" excluding a member for a pharmaceutical preparation such as a support, a release liner, and a container. For example, a content refers to, in the case of a cataplasm, the content in a base layer of the cataplasm, and in the case of a patch, the content in a pressure sensitive adhesive layer.

[(B): Organic amine]

The organic amine of Component (B) used in the present invention markedly improves the stability of Component (A) of the external preparation. Also, it improves the skin permeation of Component (A).

Preferable examples of the organic amine of Component (B) include alkanolamine. Specific examples thereof include diisopropanolamine, diethanolamine, triethanolamine chloride, triisopropanolamine, triethanolamine, trometamol, meglumine, and monoethanolamine. Among them, diisopropanolamine is preferable in the present invention.

In the external preparation of the present invention, the organic amine of Component (B) can be used singly or in combination of two or more. Although no particular limitation is imposed on the content thereof, it is preferably 0.0005 to 20% by mass, particularly preferably 0.005 to 10% by mass.

[(C): Polyoxyalkylene alkyl ether and/or polyoxyalkylene alkenyl ether]

In order to further improve the skin permeation of Component (A), a polyoxyalkylene alkyl ether and/or a polyoxyalkylene alkenyl ether is preferably added as Component (C) to the external preparation of the present invention.

The polyoxyalkylene alkyl ether and the polyoxyalkylene alkenyl ether of Component (C) refer to a product obtained by subjecting an alcohol having an alkyl group or an alkenyl group, or a phenol having an alkyl group or an alkenyl group to addition polymerization with alkylene oxide. Herein, the alcohol having an alkyl group or an alkenyl group refers to an alcohol having an alkyl group with 1 to 22 carbon atoms (a linear alkyl group with 1 to 22 carbon atoms or a branched or cyclic alkyl group with 3 to 22 carbon atoms) or an alkenyl group with 2 to 22 carbon atoms (a linear alkenyl group with 2 to 22 carbon atoms or a branched or cyclic alkenyl group with 3 to 22 carbon atoms). Examples of the alkyl group and the alkenyl group include a methyl group, an ethyl group, a propyl group, an isopropyl group, a cyclopropyl group, an allyl group, a butyl group, a pentyl group, a hexyl group, a heptyl group, an octyl group, a nonyl group, a decyl group, an isodecyl group, an undecyl group, a dodecyl group (a lauryl group), a tridecyl group, a tetradecyl group (a myristyl group), a pentadecyl group, a hexadecyl group (a cetyl group, a palmityl group), a heptadecyl group, an octadecyl group (a stearyl group), an isostearyl group, an oleyl group, a nonadecyl group, an eicosyl group, and a behenyl group. The phenol having an alkyl group or an alkenyl group refers to a phenol having the aforementioned linear, branched, or cyclic alkyl group or alkenyl group. Examples of the alkylene oxide include ethylene oxide and propylene oxide.

When subjecting the alcohol having an alkyl group or an alkenyl group or the phenol having an alkyl group or an alkenyl group to addition polymerization with alkylene oxide, as the alkylene oxide, only ethylene oxide or propylene oxide, or both ethylene oxide and propylene oxide may be addition-polymerized. The addition polymerization may be carried out based on a known method, and when both ethylene oxide and propylene oxide are addition-polymerized, either block polymerization or random polymerization may be employed. An average number of moles of the alkylene oxide added is preferably 2 to 50, more preferably 2 to 5, and particularly preferably 2 to 4.

The polyoxyalkylene alkyl ether and the polyoxyalkylene alkenyl ether of Component (C) used in the present invention can be represented by the following general formula (1):

$$R\text{-}X\text{-}O\text{-}(AO)_n\text{-}H \qquad (1)$$

wherein, R represents an alkyl group having 1 to 22 carbon atoms or an alkenyl group having 2 to 22 carbon atoms, X represents a single bond or a phenylene group, A represents an ethylene group or a propylene group, and n represents an average number of moles added of 2 to 50. An n number of A may be either one of an ethylene group and a propylene group, or a combination thereof.

In the present invention, in the aforementioned general formula (1),

(i) one in which R is an alkyl group or an alkenyl group having 8 to 22 carbon atoms, X is a single bond, and $2 \leq n \leq 5$, and
(ii) one in which R is an alkyl group having 1 to 9 carbon atoms, X is a phenylene group, and $2 \leq n \leq 5$ are preferable. Among those falling into (i) and (ii), one indicating $2 \leq n \leq 4$ is particularly preferable.

As described above, while the polyoxyalkylene alkyl ether and the polyoxyalkylene alkenyl ether of Component (C) used in the present invention can be produced based on a known method, commercially available products can also be used. Examples of the polyoxyalkylene alkyl ether and the polyoxyalkylene alkenyl ether include polyoxyethylene(2) 2-ethylhexyl ether, polyoxyethylene(4)2-ethylhexyl ether, polyoxyethylene(6)2-ethylhexyl ether, polyoxyethylene(11)2-ethylhexyl ether, polyoxyethylene(30)2-ethylhexyl ether, polyoxyethylene(3)decyl ether, polyoxyethylene(5)decyl ether, polyoxyethylene(6)decyl ether, polyoxyethylene(7)decyl ether, polyoxyethylene(10)decyl ether, polyoxyethylene(3.5) isodecyl ether, polyoxyethylene(5)isodecyl ether, polyoxyethylene(5.5)isodecyl ether, polyoxyethylene(6)isodecyl ether, polyoxyethylene(6.5)isodecyl ether, polyoxyethylene(7)isodecyl ether, polyoxyethylene(8.5)isodecyl ether, polyoxyethylene(2)lauryl ether, polyoxyethylene(2.2)lauryl ether, polyoxyethylene(3)lauryl ether, polyoxyethylene(4.2)lauryl ether, polyoxyethylene(5)lauryl ether, polyoxyethylene(6)lauryl ether, polyoxyethylene(7)lauryl ether, polyoxyethylene(7.5)lauryl ether, polyoxyethylene(8)lauryl ether, polyoxyethylene(9)lauryl ether, polyoxyethylene(10)lauryl ether, polyoxyethylene(12)lauryl ether, polyoxyethylene(13)lauryl ether, polyoxyethylene(15)lauryl ether, polyoxyethylene(19)lauryl ether, polyoxyethylene(21)lauryl ether, polyoxyethylene(25)lauryl ether, polyoxyethylene(30)lauryl ether, polyoxyethylene(40) lauryl ether, polyoxyethylene(3)tridecyl ether, polyoxyethylene(5)tridecyl ether, polyoxyethylene(6.5)tridecyl ether, polyoxyethylene(7)tridecyl ether, polyoxyethylene(7.5)tridecyl ether, polyoxyethylene(8)tridecyl ether, polyoxyethylene(8.5) tridecyl ether, polyoxyethylene(9)tridecyl ether, polyoxyethylene(10)tridecyl ether, polyoxyethylene(12)tridecyl ether, polyoxyethylene(15)tridecyl ether, polyoxyethylene(20)tridecyl ether, polyoxyethylene(3)myristyl ether, polyoxyethylene (7)myristyl ether, polyoxyethylene(12)myristyl ether, polyoxyethylene(2)cetyl ether, polyoxyethylene(5)cetyl ether, polyoxyethylene(5.5)cetyl ether, polyoxyethylene(7)cetyl ether, polyoxyethylene(8)cetyl ether, polyoxyethylene(10)cetyl ether, polyoxyethylene(13)cetyl ether, polyoxyethylene(15)cetyl ether, polyoxyethylene(20)cetyl ether, polyoxyethylene (23)cetyl ether, polyoxyethylene(25)cetyl ether, polyoxyethylene(30)cetyl ether, polyoxyethylene(40)cetyl ether, polyoxyethylene(2)stearyl ether, polyoxyethylene(3.3)stearyl ether, polyoxyethylene(4)stearyl ether, polyoxyethylene(5)stearyl ether, polyoxyethylene(7)stearyl ether, polyoxyethylene(10)stearyl ether, polyoxyethylene(11)stearyl ether, polyoxyethylene(15)stearyl ether, polyoxyethylene(20)stearyl ether, polyoxyethylene(30)stearyl ether, polyoxyethylene(50)stearyl ether, polyoxyethylene(4)isostearyl ether, polyoxyethylene(8)isostearyl ether, polyoxyethylene(12)isostearyl ether, polyoxyethylene(16)isostearyl ether, polyoxyethylene(2)oleyl ether, polyoxyethylene(4)oleyl ether, polyoxyethylene(5)oleyl ether, polyoxyethylene(6)oleyl ether, polyoxyethylene(7)oleyl ether, polyoxyethylene(8.5)oleyl ether, polyoxyethylene (9)oleyl ether, polyoxyethylene(10)oleyl ether, polyoxyethylene(11)oleyl ether, polyoxyethylene(13)oleyl ether, polyoxyethylene(13.5)oleyl ether, polyoxyethylene(14)oleyl ether, polyoxyethylene(15)oleyl ether, polyoxyethylene(20)oleyl ether, polyoxyethylene(30)oleyl ether, polyoxyethylene(40)oleyl ether, polyoxyethylene(50)oleyl ether, polyoxyethylene (5)behenyl ether, polyoxyethylene(10)behenyl ether, polyoxyethylene(20)behenyl ether, polyoxyethylene(30)behenyl ether, polyoxyethylene(3)octylphenyl ether, polyoxyethylene(6)octylphenyl ether, polyoxyethylene(8)octylphenyl ether, polyoxyethylene(10)octylphenyl ether, polyoxyethylene(15)octylphenyl ether, polyoxyethylene(20)octylphenyl ether, polyoxyethylene(40)octylphenyl ether, polyoxyethylene(5)nonylphenyl ether, polyoxyethylene(9)nonylphenyl ether, polyoxyethylene(9.5)nonylphenyl ether, polyoxyethylene(10)nonylphenyl ether, polyoxyethylene(11)nonylphenyl ether, polyoxyethylene(15)nonylphenyl ether, polyoxyethylene(18)nonylphenyl ether, polyoxyethylene(20)nonylphenyl ether, polyoxyethylene(5)polyoxypropylene(2)decyl ether, polyoxyethylene(7)polyoxypropylene(2)decyl ether, polyoxyethylene(10)polyoxypropylene(2)decyl ether, polyoxyethylene(8)polyoxypropylene(2)lauryl ether, polyoxyethylene(10)polyoxypropylene(2)lauryl ether, polyoxyethylene(13)polyoxypropylene(2)lauryl ether, polyoxyethylene(9)polyoxypropylene (2)tridecyl ether, polyoxyethylene(12)polyoxypropylene(2)tridecyl ether, polyoxyethylene(16)polyoxypropylene(2)tridecyl ether, polyoxyethylene(1)polyoxypropylene(1)cetyl ether, polyoxyethylene(1)polyoxypropylene(4)cetyl ether, polyoxyethylene(1)polyoxypropylene(8)cetyl ether, polyoxyethylene(10)polyoxypropylene(4)cetyl ether, polyoxyethylene (17)polyoxypropylene(23)cetyl ether, polyoxyethylene(20)polyoxypropylene(4)cetyl ether, polyoxyethylene(20)polyoxypropylene(8)cetyl ether, and polyoxyethylene(4)polyoxypropylene(30)stearyl ether.

Among those described above, one having an average number of moles of alkylene oxide added of 2 to 5, particularly 2 to 4, is preferable in the present invention. Among those exemplified above, preferable examples include polyoxyethylene(2)2-ethylhexyl ether, polyoxyethylene(4)2-ethylhexyl ether, polyoxyethylene(3)decyl ether, polyoxyethylene(5)decyl ether, polyoxyethylene(3.5)isodecyl ether, polyoxyethylene(5)isodecyl ether, polyoxyethylene(2)lauryl ether, polyoxyethylene(2.2)lauryl ether, polyoxyethylene(3)lauryl ether, polyoxyethylene(4.2)lauryl ether, polyoxyethylene(5)lauryl ether, polyoxyethylene(3)tridecyl ether, polyoxyethylene(5)tridecyl ether, polyoxyethylene(3)myristyl ether, polyoxyethylene(2)cetyl ether, polyoxyethylene(5)cetyl ether, polyoxyethylene(2)stearyl ether, polyoxyethylene(3.3) stearyl ether, polyoxyethylene(4)stearyl ether, polyoxyethylene(5)stearyl ether, polyoxyethylene(4)isostearyl ether, polyoxyethylene(2)oleyl ether, polyoxyethylene(4)oleyl ether, polyoxyethylene(5)oleyl ether, polyoxyethylene(5)behenyl ether, polyoxyethylene(3)octylphenyl ether, polyoxyethylene(5)nonylphenyl ether, polyoxyethylene(1)polyoxypropylene (1)cetyl ether, and polyoxyethylene(1)polyoxypropylene(4)cetyl ether. Also, particularly preferable examples include polyoxyethylene(2)2-ethylhexyl ether, polyoxyethylene(4)2-ethylhexyl ether, polyoxyethylene(3)decyl ether, polyoxyethylene(3.5)isodecyl ether, polyoxyethylene(2)lauryl ether, polyoxyethylene(2.2)lauryl ether, polyoxyethylene(3)lauryl ether, polyoxyethylene(3)tridecyl ether, polyoxyethylene(3)myristyl ether, polyoxyethylene(2)cetyl ether, polyoxyethylene(2)stearyl ether, polyoxyethylene(3.3)stearyl ether, polyoxyethylene(4)stearyl ether, polyoxyethylene(4)isostearyl ether, polyoxyethylene(2)oleyl ether, polyoxyethylene(4)oleyl ether, polyoxyethylene(3)octylphenyl ether, and polyoxyethylene(1)polyoxypropylene(1)cetyl ether.

In the external preparation of the present invention, the polyoxyalkylene alkyl ether and/or the polyoxyalkylene alkenyl ether of Component (C) can be used singly or in combination of two or more. Although no particular limitation is imposed on the content thereof, the content is preferably 0.01 to 50% by mass, more preferably 0.05 to 30% by mass, even more preferably 0.1 to 28% by mass, and particularly preferably 0.1 to 25% by mass.

[(D): Terpene and/or essential oil containing terpene]

In order to further improve the skin permeation of Component (A), a terpene and/or an essential oil containing a terpene can be added as Component (D) to the external preparation of the present invention. Although no particular limitation is imposed on the terpene and/or the essential oil containing a terpene, examples thereof can include monoterpene, sesquiterpene, and/or an essential oil containing these terpenes. Examples of the terpene include isoborneol, irone, ocimene, carveol, carvotanacetone, carvomenthone, carvone, carene, carone, camphene, camphor, geraniol, cymene, sabinene, safranal, cyclocitral, citral, citronellal, citronellic acid, citronellol, cineole, sylvestrene, thujyl alcohol, thujone, terpineol, terpinene, terpinolene, tricyclene, nerol, pinene, pinocampheol, pinol, piperitenone, phellandral, phellandrene, fenchene, fenchyl alcohol, perillyl alcohol, perillyl aldehyde, borneol, myrcene, menthol, menthone, ionol, ionone, linalool, and limonene. These terpenes include a single stereoisomer and a mixture thereof. Also, examples of the essential oil containing a terpene include anise oil, ylang-ylang oil, orris oil, fennel oil, orange oil, cananga oil, chamomile oil, cajuput oil, caraway oil, cubeb oil, grapefruit oil, cinnamon oil, coriander oil, saffron oil, zanthoxylum fruit oil, perilla oil, citriodora oil, citronella oil, ginger oil, cardamom oil, camphor oil, ginger glass oil, spearmint oil, peppermint oil, geranium oil, star aniseed oil, clove oil, turpentine oil, bitter orange peel oil, neroli oil, basil oil, mentha oil, palmarosa oil, pimento oil, petitgrain oil, bay oil, pennyroyal oil, chenopodium oil, bergamot oil, bois de rose oil, hosho oil, majoran oil, mandarin oil, melissa oil, eucalyptus oil, lime oil, lavender oil, linaloe oil, lemon oil, lemonglass oil, rose oil, rosemary oil, and Roman chamomile oil.

In the present invention, preferable examples of the terpene include camphor, d-camphor, dl-camphor, geraniol, citronellal, terpineol, borneol, d-borneol, menthol, dl-menthol, l-menthol, and limonene, and menthol, dl-menthol, and l-menthol are particularly preferable. Also, preferable examples of the essential oil containing a terpene include ylang-ylang oil, fennel oil, orange oil, chamomile oil, cinnamon oil, perilla oil, citronella oil, ginger oil, camphor oil, peppermint oil, geranium oil, clove oil, turpentine oil, bitter orange peel oil, neroli oil, mentha oil, palmarosa oil, bergamot oil, eucalyptus oil, lavender oil, linaloe oil, lemon oil, rose oil, rosemary oil, and Roman chamomile oil, and mentha oil is particularly preferable.

In the external preparation of the present invention, the terpene and/or the essential oil containing a terpene of Component (D) can be used singly or in combination of two or more. Although no particular limitation is imposed on the content thereof, it is preferably 0.0001 to 20% by mass, more preferably 0.001 to 15% by mass, and particularly preferably 0.005 to 10% by mass.

[(E): Higher alcohol]

In order to further improve the skin permeation of Component (A), a higher alcohol can be added as Component (E) to the external preparation of the present invention. Although no particular limitation is imposed on the higher alcohol, examples thereof can include a saturated or unsaturated aliphatic alcohol having 8 to 22 carbon atoms. Specific examples thereof include a linear or branched, saturated or unsaturated aliphatic alcohol such as octyl alcohol, nonyl alcohol, decyl alcohol, isodecyl alcohol, undecyl alcohol, lauryl alcohol, tridecyl alcohol, myristyl alcohol, pentadecyl alcohol, cetyl alcohol, heptadecyl alcohol, stearyl alcohol, isostearyl alcohol, oleyl alcohol, linoleyl alcohol, nonadecyl alcohol, eicosyl alcohol, and behenyl alcohol.

In the present invention, a saturated or unsaturated aliphatic alcohol having 8 to 20 carbon atoms, particularly 8 to 18 carbon atoms, is more preferable. Among those aliphatic alcohols, octyl alcohol, nonyl alcohol, decyl alcohol, isodecyl alcohol, undecyl alcohol, lauryl alcohol, tridecyl alcohol, isostearyl alcohol, oleyl alcohol, linoleyl alcohol, and the like are particularly preferable.

In the external preparation of the present invention, the higher alcohol of Component (E) can be used singly or in combination of two or more. Although no particular limitation is imposed on the content thereof, it is preferably 0.0001 to 30% by mass, more preferably 0.001 to 20% by mass, and particularly preferably 0.005 to 15% by mass.

[Dosage form, excipient]

Although no particular limitation is imposed on the dosage form of the external preparation of the present invention, examples thereof include ones listed in the general rules for preparations in The Japanese Pharmacopoeia, fifteenth edition, such as a liquid preparation, a gel, an ointment, a cream, a gel cream, a cataplasm, a patch, a liniment, a lotion, a transdermal system, and an aerosol. They can be produced by a known method. In the production of these dosage forms, excipients such as a pH adjuster, an antioxidant, a surfactant, an ultraviolet ray absorber, and a percutaneous absorption enhancer may also be added in addition to the essential components of the present invention.

Examples of the percutaneous absorption enhancer include a fatty acid such as caprylic acid, capric acid, caproic acid, lauric acid, myristic acid, palmitic acid, stearic acid, isostearic acid, oleic acid, linoleic acid, linolenic acid, and a salt of these fatty acids; and a fatty acid ester such as hexyl laurate, isopropyl myristate, cetyl myristate, isocetyl myristate, myristyl myristate, octyldodecyl myristate, isopropyl palmitate, ethylhexyl palmitate, cetyl palmitate, ethyl stearate, isocetyl stearate, stearyl stearate, ethyl isostearate, isopropyl isostearate, hexyldecyl isostearate, isostearyl isostearate, ethyl oleate, decyl oleate, oleyl oleate, ethyl linoleate, and isopropyl linoleate.

Examples of the antioxidant include sodium sulfite, dried sodium sulfite, dibutylhydroxytoluene, thymol, tocopherol, tocopherol acetate, butylhydroxyanisole, propyl gallate, and 2-mercaptobenzimidazole.

When the external preparation of the present invention contains, as Component (A), a non-steroidal analgesic/anti-inflammatory agent containing a carboxyl group in the chemical structure (for example, acemetacin, amfenac sodium, ibuprofen, indometacin, indometacin farnesil, etodolac, ketoprofen, zaltoprofen, diclofenac sodium, sulindac, tiaprofenic acid, piroxicam, felbinac, pranoprofen, flurbiprofen, flurbiprofen axetil, mefenamic acid, loxoprofen sodium, and the like), and as Component (D), menthol and/or an essential oil containing menthol, a known problem is that a reaction between the carboxyl group of the non-steroidal analgesic/anti-inflammatory agent and menthol produces a menthol ester form, resulting in a reduced content of the non-steroidal analgesic/anti-inflammatory agent in the external preparation. For this, a fatty acid metal salt such as zinc undecylenate, zinc stearate, aluminum stearate, calcium stearate, magnesium stearate, sodium stearate, zinc palmitate, zinc myristate, magnesium myristate, zinc laurate, and sodium laurate, a metal oxide such as zinc oxide, calcium oxide, titanium oxide, and magnesium oxide, and a metal hydroxide such as aluminum hydroxide, potassium hydroxide, calcium hydroxide, and sodium hydroxide may be added.

Further, considering the skin permeation of Component (A), triethylene glycol may further be added to the external preparation of the present invention.

Also, when the non-steroidal analgesic/anti-inflammatory agent of Component (A) is amfenac sodium, one or two or more kinds selected from the group consisting of magnesium oxide, magnesium carbonate, and calcium carbonate are preferably added in terms of improving temporal stability of amfenac sodium.

[Cataplasm]

A case in which the dosage form is a cataplasm will be described. A cataplasm has a structure of a support, a cataplasm base layer, and a release liner, laminated in this order. The essential components of the present invention are contained in the cataplasm base layer. The support is not particularly limited and a known support may be used, and examples thereof include nonwoven fabric and knitted fabric such as polyethylene, polypropylene, polyester, nylon, and rayon.

Likewise, a known cataplasm base may be used as the cataplasm base and no particular limitation is imposed thereon, and examples thereof include one or two or more kinds selected from the group consisting of polyacrylic acid, sodium polyacrylate, partially neutralized polyacrylate, an n-vinyl acetamide-sodium acrylate copolymer, polyvinyl alcohol, polyvinyl pyrrolidone, hydroxymethylcellulose, carboxymethylcellulose sodium, alginic acid, sodium alginate, gelatin, acacia, and the like, and one obtained by crosslinking the above substance with a salt of metal such as aluminum, zinc, magnesium, and calcium. In addition to the cataplasm base and the essential components of the present invention, Components (C) to (E) described above, and an excipient, such as a filler such as kaolin, talc, and titanium oxide, and a percutaneous absorption enhancer, can be added to the cataplasm base layer as desired. For amfenac sodium being unstable in an acidic condition, when the non-steroidal analgesic/anti-inflammatory agent of Component (A) is amfenac sodium, pH of the cataplasm base layer is preferably adjusted to 6.5 to 9.

Likewise, a known release liner may be used as the release liner and no particular limitation is imposed thereon, and examples thereof include a film such as polyester, polyethylene, polypropylene, an ethylene-vinyl acetate copolymer, and cellophane.

The cataplasm can be produced by, based on a known method, spreading a cataplasm base layer prepared by adding the essential components of the present invention, if desired, further addition of Components (C), (D) and/or Component (E) and other excipients, over a support or a release liner, and then attaching the release liner or the support on the thus-obtained cataplasm base layer.

A ratio of the cataplasm base layer to the total amount of the cataplasm in the cataplasm of the present invention is preferably 50 to 99% by mass, more preferably 60 to 99% by mass, and particularly preferably 70 to 95% by mass.

Preferable contents of Components (A) and (B), and desired Components (C), (D) and (E) in the cataplasm base layer are as described above, and the content of the cataplasm base in the cataplasm base layer is preferably 1 to 60% by mass, more preferably 10 to 55% by mass, and particularly preferably 20 to 50% by mass.

[Patch]

Then, a case in which the dosage form is a patch will be described. A patch has a structure of a support, a pressure sensitive adhesive layer, and a release liner, laminated in this order. The essential components of the present invention are contained in the pressure sensitive adhesive layer. The support is not particularly limited and a known support may be used, and examples thereof include paper, fabric, nonwoven fabric as well as a single layer film of polyester, polyethylene, polypropylene, polybutadiene, polyurethane, polyvinyl acetate, nylon, polyvinylidene chloride, and the like, and a laminate of these materials.

Likewise, a known pressure sensitive adhesive may be used as the pressure sensitive adhesive and no particular limitation is imposed thereon, and examples thereof include an acrylic adhesive, a synthetic rubber adhesive, and a natural rubber adhesive. These pressure sensitive adhesives can be used singly or in combination of two or more. These pressure sensitive adhesives can also be emulsified.

Examples of the acrylic adhesive include a polymer composed of monomers of acrylic acid, sodium acrylate, and methacrylic acid as well as alkyl (meth)acrylate such as methyl acrylate, ethyl acrylate, butyl acrylate, octyl acrylate, isononyl acrylate, 2-ethylhexyl acrylate, methyl methacrylate, butyl methacrylate, 2-ethylhexyl methacrylate, hydroxyethyl methacrylate, and dodecyl methacrylate, a copolymer composed of two or more kinds of the above monomers, and a copolymer of alkyl (meth)acrylate and a vinyl compound such as vinyl acetate, vinyl propionate, styrene, and N-vinyl-2-pyrrolidone (an alkyl (meth)acrylate-vinyl compound copolymer). These acrylic adhesives can be used singly or in combination of two or more. Specific examples thereof include an acrylic acid-octyl acrylate copolymer, an acrylates-vinyl acetate copolymer, a 2-ethylhexyl acrylate-vinyl pyrrolidone copolymer solution, a 2-ethylhexyl acrylate-2-ethylhexyl methacrylate-dodecyl methacrylate copolymer solution, an ethyl acrylate-methyl methacrylate copolymer dispersion, an emulsion of methyl acrylate and 2-ethylhexyl acrylate copolymer resin, an acrylic resin alkanolamine solution, a methacrylic acid-n-butyl acrylate copolymer, a silkfibroin acrylate copolymer, starch grafted acrylate 300, starch grafted acrylate 1000, a butyl acrylate-2-ethylhexyl methacrylate-diacetone acrylamide copolymer, a butyl acrylate-2-hydroxyethyl methacrylate-diacetone acrylamide copolymer, a butyl acrylate-ethyl acrylate-2-hydroxyethyl methacrylate-diacetone acrylamide copolymer, a butyl acrylate-2-ethylhexyl acrylate-2-hydroxyethyl methacrylate-diacetone acrylamide copolymer, an isononyl acrylate-2-hydroxyethyl methacrylate-diacetone acrylamide copolymer, a 2-ethylhexyl acrylate-2-hydroxyethyl methacrylate-diacetone acrylamide copolymer, a butyl acrylate-ethyl acrylate-3-hydroxypropyl methacrylate-2-hydroxyethyl methacrylate-diacetone acrylamide copolymer, and a butyl acrylate-ethyl acrylate-3-hydroxypropyl methacrylate-diacetone acrylamide copolymer. It is to be noted that, when the non-steroidal analgesic/anti-inflammatory agent is amfenac sodium, a copolymer composed of monomers of diacetone acrylamide is preferably used as the acrylic adhesive from the viewpoints of the stability and the drug release property of amfenac sodium.

Examples of the synthetic rubber adhesive include cis isoprene rubber, styrene isoprene gum, cis polyisoprene rubber, polyisoprene gum, styrene butadiene rubber, a styrene-isoprene-styrene block copolymer, a styrene-butadiene-styrene block copolymer, polyisoprene, polyisobutylene, chloroprene rubber, polybutene, and styrene butadiene rubber latex. These synthetic rubber adhesives can be used singly or in combination of two or more.
Examples of the natural rubber adhesive include acacia and natural rubber latex. These natural rubber adhesives can be used singly or in combination of two or more.

In addition to the pressure sensitive adhesive and the essential components of the present invention, Components (C) to (E) described above, and excipients such as a plasticizer, a tackifier resin, a filler, an ultraviolet ray absorber, a percutaneous absorption enhancer, an antioxidant, and a water-soluble/water-swellable polymer can be added, as desired, to the pressure sensitive adhesive layer.

Examples of the plasticizer include liquid paraffin, light liquid paraffin, cetyl octanoate, hexyl laurate, isopropyl myristate, octyldodecyl myristate, isopropyl palmitate, butyl stearate, myristyl lactate, dioctyl adipate, diethyl sebacate, diisopropyl sebacate, dioctyl sebacate, diisopropyl adipate, dioctyl succinate, octyldodecanol, hexyldecanol, almond oil, olive oil, camellia oil, castor oil, peanut oil, mentha oil, l-menthol, diethylene glycol, propylene glycol, dipropylene glycol, polyethylene glycol, polypropylene glycol, triacetin, and triethyl citrate. These plasticizers can be used singly or in combination of two or more.

Examples of the tackifier resin include rosin, hydrogenated rosin glycerol ester, ester gum, maleated rosin glycerol ester, terpene resin, petroleum resin, alicyclic saturated hydrocarbon resin, and aliphatic hydrocarbon resin. These tackifier resins can be used singly or in combination of two or more.

Examples of the filler include zinc oxide, aluminum oxide, titanium dioxide, magnesium oxide, iron oxide, zinc stearate, calcium carbonate, and silica. These fillers can be used singly or in combination of two or more.

Examples of the water-soluble/water-swellable polymer include a carboxy vinyl polymer, fully hydrolyzed polyvinyl alcohol, partially hydrolyzed polyvinyl alcohol, povidone, methylcellulose, hydroxyethylcellulose, carboxymethylethylcellulose, carmellose, carmellose potassium, carmellose calcium, carmellose sodium, hydroxypropylcellulose, hydroxypropylmethylcellulose, sodium alginate, propylene glycol alginate, carboxymethyl starch sodium, xanthan gum, dextran, and dextrin. These water-soluble/water-swellable polymers can be used singly or in combination of two or more. Among them, fully hydrolyzed polyvinyl alcohol, partially hydrolyzed polyvinyl alcohol, povidone, hydroxypropylcellulose, and hydroxypropylmethylcellulose are preferable.

Likewise, a known release liner may be used as the release liner and no particular limitation is imposed thereon, and examples thereof include polyester, polyethylene, polypropylene, an ethylene-vinyl acetate copolymer, and a film such as cellophane.

The patch can be produced based on a known method (such as the solvent method, the hot melt method, and the emulsion method). For example, the essential components of the present invention, the pressure sensitive adhesive layer component, and Components (C), (D) and (E), and excipients, as desired, are immersed in an appropriate organic solvent and uniformly dispersed by stirring to produce an adhesive layer solution. Then, the resulting adhesive layer solution is spread over a support or a release liner, the solvent is dried by volatilization, and the release liner or the support is attached thereon, whereby the patch can be produced. The patch can also be produced by applying the pressure sensitive adhesive layer component to a support or a release liner and spreading it by a knife coater, and then attaching the release liner or the support thereon.

When the non-steroidal analgesic/anti-inflammatory agent of Component (A) is in the form of a salt such as amfenac sodium, considering the percutaneous absorbability, carboxylic acid such as citric acid, succinic acid, tartaric acid, maleic acid, fumaric acid, salicylic acid, and acetic acid may be further mixed in the pressure sensitive adhesive layer.

Amfenac sodium is unstable in an acidic condition. For this, when the non-steroidal analgesic/anti-inflammatory agent of Component (A) is amfenac sodium and the patch is produced by the emulsion method, pH is preferably adjusted to 6.5 to 9 during the production process of the pressure sensitive adhesive layer.

Further, when an acrylic adhesive is used as the pressure sensitive adhesive, magnesium oxide is preferably added to the adhesive layer in terms of improving temporal stability of amfenac sodium. In this case, the amount of magnesium oxide mixed in may be equal to or less than an equivalent of amfenac sodium.

Although no particular limitation is imposed on a ratio of the pressure sensitive adhesive layer in the patch of the present invention, it is preferably 1 to 70% by mass, more preferably 10 to 60% by mass, and particularly preferably 25 to 50% by mass with respect to the total amount of the patch.

Preferable contents of Components (A) and (B), and desired Components (C), (D) and (E) in the pressure sensitive adhesive layer are as described above, and the content of pressure sensitive adhesive in the pressure sensitive adhesive layer is preferably 50 to 99% by mass, more preferably 60 to 99% by mass, and particularly preferably 70 to 95% by mass.

[Liquid preparation]

When the dosage form of the external preparation of the present invention is, for example, a liquid preparation, it may be produced based on a known method, using a solvent permitted to be used as a liquid preparation for external application. Although no particular limitation is imposed on the solvent, the liquid preparation can be produced by, for example, dissolving the essential components of the present invention in one or two or more kinds selected from the group consisting of lower alcohol such as methanol, ethanol, propanol, and isopropanol, polyhydric alcohol such as ethylene glycol, propylene glycol, isopropylene glycol, and 1,3-butylene glycol, water, and the like with appropriate addition of Components (C), (D) and (E), and excipients such as a pH adjuster, an antioxidant, a surfactant, an ultraviolet ray absorber, and a percutaneous absorption enhancer, as desired. For amfenac sodium being unstable in an acidic condition, when the non-steroidal analgesic/anti-inflammatory agent is amfenac sodium, pH of the liquid preparation is preferably adjusted to 6.5 to 9.

[Ratio of each component]

While the content of each of Components (A) and (B), and desired Components (C), (D) and (E) in the external preparation of the present invention is as described above, as for the relationship among the contents of each component, the contents of the components are preferably in the following ratios within the range of the contents described above.

That is, a ratio of the content of Component (B) to one part by mass of the content of Component (A) is preferably 0.001 to 20 parts by mass, more preferably 0.005 to 10 parts by mass, and particularly preferably 0.001 to 5 parts by mass.

Further, a ratio of the content of Component (C) to one part by mass of the content of Component (A) is preferably 0.01 to 20 parts by mass, more preferably 0.05 to 15 parts by mass, and particularly preferably 0.1 to 11 parts by mass.

Moreover, a ratio of the content of Component (D) to one part by mass of the content of Component (A) is preferably 0.01 to 10 parts by mass, more preferably 0.1 to 9 parts by mass, and particularly preferably 0.25 to 8 parts by mass.

Further, a ratio of the content of Component (E) to one part by mass of the content of Component (A) is preferably 0.01 to 20 parts by mass, more preferably 0.05 to 15 parts by mass, and particularly preferably 0.1 to 10 parts by mass.

[Examples]

Hereinbelow, the present invention will be more specifically described with reference to Examples; however, the present invention is not limited by these Examples.

Example 1 Patch

In 100.0 g of ethyl acetate, 30.0 g of a styrene-isoprene-styrene block copolymer (SIS5505P: JSR Corporation), 24.0 g of terpene resin (YS resin PX1150N: Yasuhara Chemical Co., Ltd.), 20.0 g of polybutene (Polybutene 3SH: NOF Corporation), and 10.5 g of light liquid paraffin (HICALL M72: Kaneda Corporation) were dissolved to give an adhesive phase.

And then, 1.0 g of amfenac sodium was added to 5.0 g of polyoxyethylene(2)lauryl ether (NIKKOL BL-2: Nihon Surfactant Kogyo K.K), and after confirmation of dissolution, 0.5 g of diisopropanolamine (diisopropanolamine: Mitsui Fine Chemical Inc.), 5.0 g of oleyl alcohol (NOVOL J: Croda Japan K.K), and 4.0 g of l-menthol (l-menthol (menthol): The Suzuki Menthol Co., Ltd.) were added. The adhesive phase prepared in advance was then added, and the resulting mixture was thoroughly mixed to give a drug solution.

The drug solution thus obtained was spread over a PET film which had been subjected to silicone treatment by a plaster production apparatus, and before an adhesive layer cooled down, the resulting PET film was laminated with a support (knitted material: TV-105: Japan Vilene Company, Ltd.) to give a patch containing 1% by mass of amfenac sodium.

Example 2 Patch

Except for changing the amounts of light liquid paraffin and diisopropanolamine to 10.0 g and 1.0 g, respectively, a patch containing 1% by mass of amfenac sodium was obtained in the same manner as Example 1.

Example 3 patch

Except for changing the amounts of light liquid paraffin and diisopropanolamine to 9.5 g and 1.5 g, respectively, a patch containing 1% by mass of amfenac sodium was obtained in the same manner as Example 1.

Reference Example 1

Except for changing the amount of the light liquid paraffin to 11.0 g and not adding diisopropanolamine, a patch containing 1% by mass of amfenac sodium was obtained in the same manner as Example 1.

Examples 4 to 9 Patch

Based on the blend components and the amounts blended shown in Table 1, patches were produced in the same manner as Example 1.

[Table 1]

|  | Example 4 | Example 5 | Example 6 | Example 7 | Example 8 | Example 9 |
|---|---|---|---|---|---|---|
| SIS | 30.0g | 30.0g | 30.0g | 30.0g | 30.0g | 30.0g |
| Light liquid paraffin | 10.0g | 10.0g | 9.5g | 9.5g | 9.0g | 8.5g |
| Polybutene | 20.0g | 20.0g | 20.0g | 20.0g | 20.0g | 20.0g |
| Terpene resin | 24.0g | 24.0g | 24.0g | 24.0g | 24.0g | 24.0g |
| Amfenac sodium | 0.5g | 1.5g | 1.5g | 2.0g | 2.0g | 2.0g |
| Lauromacrogol | 5.0g | 5.0g | 5.0g | 5.0g | 5.0g | 5.0g |
| Oleyl alcohol | 5.0g | 5.0g | 5.0g | 5.0g | 5.0g | 5.0g |
| 1-Menthol | 4.0g | 4.0g | 4.0g | 4.0g | 4.0g | 4.0g |
| Diisopropanolamine | 1.5g | 0.5g | 1.0g | 0.5g | 1.0g | 1.5g |
| SIS: styrene-isoprene-styrene block copolymer | | | | | | |

Example 10 Patch

After mixing 13.0 g of a styrene-isoprene-styrene block copolymer (SIS5002: JSR Corporation), 38.0 g of alicyclic saturated hydrocarbon resin (ARKON P-100: Arakawa Chemical Industries Ltd.), 5.0 g of polyisobutylene (Himol 5H: Nippon Petroleum Refining Co., Ltd.), and 35.85 g of liquid paraffin (HICALL M352: Kaneda Corporation), the resulting mixture was melted at 150°C to give an adhesive phase.

And then, 2.0 g of amfenac sodium was added to 1.0 g of polyoxyethylene(2)lauryl ether (NIKKOL BL-2: Nihon Surfactant Kogyo K.K), and after confirmation of dissolution, 0.15 g of diisopropanolamine (diisopropanolamine: Mitsui Fine Chemical Inc.), 0.5 g of dibutylhydroxytoluene (Yoshinox BHT: API Corporation), 3.0 g of Macrogol 400 (Macrogol 400: NOF Corporation), 0.5 g of oleyl alcohol (NOVOL J: Croda Japan K.K), and 1.0 g of l-menthol (l-menthol (menthol): The Suzuki Menthol Co., Ltd.) were added. The adhesive phase prepared in advance was then added, and the resulting mixture was thoroughly mixed to give a drug solution.

The drug solution thus obtained was spread over a PET film which had been subjected to silicone treatment by a plaster production apparatus, and before a adhesive layer cooled down, the resulting PET film was laminated with a support (knitted material: TV-105: Japan Vilene Company, Ltd.) to give a patch containing 2% by mass of amfenac sodium.

Test Example 1

The patches prepared in Examples 1 to 3 and Reference Example 1 were stored at 60°C for 3 days and for 1 week, and the ratio of remaining amfenac after storage was measured by an HPLC method. The results were shown in Table 2.

[Table 2]

|  | 60°C for 3 days | 60°C for 1 week |
|---|---|---|
| Reference Example 1 | 89.2% | 85.9% |
| Example 1 | 96.5% | 93.9% |
| Example 2 | 98.7% | 96.8% |
| Example 3 | 101.1% | 100.5% |

As apparent from Table 2, the stability of amfenac in the external preparation was improved in the patches containing an organic amine. Further, it was also revealed that the stability of amfenac was improved in a manner dependent on the concentration of the organic amine.

Test Example 2

The percutaneous absorbability of the patches prepared in Examples 2, 6 and 8 was measured by the following method.

The patch was used as a donor, and a solution of Macrogol 400 / physiological saline (5/5) + 0.01% sodium dodecyl sulfate was used as a receptor solution. The skin excised from the abdomen of a Wistar rat (male, 8 weeks old) was used as a permeation membrane. The skin was immobilized on a permeation unit of a vertical diffusion cell (Frantz cell) with the stratum corneum side facing the donor side. One sheet of the patch (2 cm in diameter) was set in the donor side, and the receptor side was filled with 31 mL of the receptor solution. Keeping the vertical diffusion cell at 32°C, the permeation experiment was carried out. In order to prevent peeling off of a pharmaceutical preparation during the experiment, steel balls, No. 3 (7 balls: 7.3 g), were placed as a weight. Further, in order to prevent evaporation of water and the like during experimentation, the sampling port was covered with a film (PARAFILM; the product of American National Can).

Two, four, six, and eight hours after initiation, 1 mL of the receptor solution was sampled from the sampling port, and the drug concentration in the receptor solution was quantitated by HPLC using an ODS column. The amount of drug permeated per unit area ($\mu$g/cm$^2$) was calculated from the measurement value. Also, a permeability coefficient (cm/h) was calculated by the following formula based on the values measured after four to eight hours. The results thus obtained are shown in Table 3.

    (Permeability coefficient) = slope (values measured after

    four to eight hours) / (amfenac sodium concentration in

    the pharmaceutical preparation)

Test Example 3

The patches obtained in Examples 2, 6 and 8 were evaluated for their appearance. The appearance was evaluated by visual observation of the color tone of the adhesive layer right after production. The results thus obtained are shown in Table 3.

[Table 3]

|  | Amount of drug permeated after 8 hours ($\mu$g/cm$^2$) | Appearance at the time of production |
|---|---|---|
| Example 2 | 6.8 | Light yellow |
| Example 6 | 25.1 | Light yellow |
| Example 8 | 44.6 | Light yellow |

As apparent from Table 3, it was revealed that skin permeation of the external preparation of the present invention in which an organic amine was mixed was improved in a manner dependent on the concentration of a non-steroidal analgesic/anti-inflammatory agent, and the external preparation also had excellent appearance.

## Claims

1. An external preparation comprising the following components (A) and (B):

   (A) a non-steroidal analgesic/anti-inflammatory agent, and
   (B) an organic amine.

2. The external preparation according to claim 1, wherein (B) the organic amine is alkanolamine.

3. The external preparation according to claim 2, wherein (B) the organic amine is selected from the group consisting of diisopropanolamine, diethanolamine, triethanolamine chloride, triisopropanolamine, triethanolamine, trometamol, meglumine, and monoethanolamine.

4. The external preparation according to any one of claims 1 to 3, further comprising (C) a polyoxyalkylene alkyl ether and/or a polyoxyalkylene alkenyl ether.

5. The external preparation according to claim 4, wherein (C) the polyoxyalkylene alkyl ether and the polyoxyalkylene alkenyl ether are represented by the formula (1):

$$R\text{-}X\text{-}O\text{-}(AO)_n\text{-}H \qquad (1)$$

   wherein, R represents an alkyl group having 1 to 22 carbon atoms or an alkenyl group having 2 to 22 carbon atoms, X represents a single bond or a phenylene group, A represents an ethylene group or a propylene group, and n represents an average number of moles added of 2 to 50, and an n number of A can be either one of an ethylene group and a propylene group, or a combination thereof.

6. The external preparation according to claim 5, wherein the n in the formula (1) is 2 to 5.

7. The external preparation according to any one of claims 1 to 6, further comprising (D) a terpene and/or an essential oil containing a terpene.

8. The external preparation according to claim 7, wherein (D) the terpene is selected from the group consisting of isoborneol, irone, ocimene, carveol, carvotanacetone, carvomenthone, carvone, carene, carone, camphene, camphor, geraniol, cymene, sabinene, safranal, cyclocitral, citral, citronellal, citronellic acid, citronellol, cineole, sylvestrene, thujyl alcohol, thujone, terpineol, terpinene, terpinolene, tricyclene, nerol, pinene, pinocampheol, pinol, piperitenone, phellandral, phellandrene, fenchene, fenchyl alcohol, perillyl alcohol, perillyl aldehyde, borneol, myrcene, menthol, menthone, ionol, ionone, linalool, and limonene.

9. The external preparation according to claim 7, wherein (D) the essential oil comprising a terpene is selected from the group consisting of anise oil, ylang-ylang oil, orris oil, fennel oil, orange oil, cananga oil, chamomile oil, cajuput oil, caraway oil, cubeb oil, grapefruit oil, cinnamon oil, coriander oil, saffron oil, zanthoxylum fruit oil, perilla oil, citriodora oil, citronella oil, ginger oil, cardamom oil, camphor oil, ginger glass oil, spearmint oil, peppermint oil, geranium oil, star aniseed oil, clove oil, turpentine oil, bitter orange peel oil, neroli oil, basil oil, mentha oil, palmarosa oil, pimento oil, petitgrain oil, bay oil, pennyroyal oil, chenopodium oil, bergamot oil, bois de rose oil, hosho oil, majoran oil, mandarin oil, melissa oil, eucalyptus oil, lime oil, lavender oil, linaloe oil, lemon oil, lemonglass oil, rose oil, rosemary oil, and Roman chamomile oil.

10. The external preparation according to any one of claims 1 to 9, further comprising (E) a higher alcohol.

11. The external preparation according to claim 10, wherein (E) the higher alcohol is a saturated or unsaturated aliphatic alcohol having 8 to 22 carbon atoms.

12. The external preparation according to claims 10, wherein (E) the higher alcohol is selected from the group consisting of octyl alcohol, nonyl alcohol, decyl alcohol, isodecyl alcohol, undecyl alcohol, lauryl alcohol, tridecyl alcohol, myristyl alcohol, pentadecyl alcohol, cetyl alcohol, heptadecyl alcohol, stearyl alcohol, isostearyl alcohol, oleyl alcohol, linoleyl alcohol, nonadecyl alcohol, eicosyl alcohol, and behenyl alcohol.

13. The external preparation according to any one of claims 1 to 12, wherein (A) the non-steroidal analgesic/anti-inflammatory agent is selected from the group consisting of actarit, acemetacin, ampiroxicam, amfenac, ibuprofen, indometacin, etodolac, ketoprofen, zaltoprofen, diclofenac, sulindac, celecoxib, tiaprofenic acid, tenoxicam, naproxen, piroxicam, felbinac, pranoprofen, flurbiprofen, mefenamic acid, medicoxib, meloxicam, mofezolac, refecoxib, loxoprofen, lobenzarit, lornoxicam, and a salt of these substances.

14. The external preparation according to any one of claims 1 to 13, wherein (A) the non-steroidal analgesic/anti-inflammatory agent is amfenac or a salt thereof.

15. The external preparation according to any one of claims 1 to 14, wherein a dosage form of the external preparation is a liquid preparation, a gel, an ointment, a cream, a gel cream, a cataplasm, a patch, a liniment, a lotion, a transdermal system, or an aerosol.

<table>
<tr><td colspan="2"><b>INTERNATIONAL SEARCH REPORT</b></td><td>International application No.<br>PCT/JP2010/001761</td></tr>
</table>

A. CLASSIFICATION OF SUBJECT MATTER
*A61K45/00*(2006.01)i, *A61K9/06*(2006.01)i, *A61K9/08*(2006.01)i, *A61K9/12*(2006.01)i, *A61K9/70*(2006.01)i, *A61K31/196*(2006.01)i, *A61K47/06*(2006.01)i, *A61K47/08*(2006.01)i, *A61K47/10*(2006.01)i, *A61K47/18*(2006.01)i,

According to International Patent Classification (IPC) or to both national classification and IPC

B. FIELDS SEARCHED

Minimum documentation searched (classification system followed by classification symbols)
A61K45/00, A61K9/06, A61K9/08, A61K9/12, A61K9/70, A61K31/196, A61K47/06, A61K47/08, A61K47/10, A61K47/18, A61K47/34, A61P29/00

Documentation searched other than minimum documentation to the extent that such documents are included in the fields searched
Jitsuyo Shinan Koho        1922–1996   Jitsuyo Shinan Toroku Koho   1996–2010
Kokai Jitsuyo Shinan Koho  1971–2010   Toroku Jitsuyo Shinan Koho   1994–2010

Electronic data base consulted during the international search (name of data base and, where practicable, search terms used)
JSTPlus/JMEDPlus/JST7580(JDreamII), CA(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2008/093686 A1  (Medrx Co., Ltd.),<br>07 August 2008 (07.08.2008),<br>each claim; paragraphs [0014], [0017] to [0020],<br>[0027] to [0028], [0032] to [0033], [0041]<br>& US 2010/0029704 A    & EP 2128123 A1 | 1-13,15<br>14 |
| X<br>Y | JP 2003-306430 A  (Lion Corp.),<br>28 October 2003 (28.10.2003),<br>each claim; paragraphs [0012] to [0015], [0025],<br>[0031], [0033], [0044]<br>(Family: none) | 1-13,15<br>14 |
| X<br>Y<br>A | JP 2004-83462 A  (Yutoku Pharmaceutical Ind.<br>Co., Ltd.),<br>18 March 2004 (18.03.2004),<br>each claim; paragraphs [0019] to [0030]<br>(Family: none) | 1-8,10,13,15<br>14<br>9,12 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>24 March, 2010 (24.03.10) | Date of mailing of the international search report<br>06 April, 2010 (06.04.10) |
|---|---|
| Name and mailing address of the ISA/<br>  Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2010/001761 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y<br>A | WO 2009/028650 A1 (Yutoku Pharmaceutical Ind.<br>Co., Ltd.),<br>05 March 2009 (05.03.2009),<br>claim 40; paragraphs [0020] to [0021]<br>(Family: none) | 14<br>1-13,15 |
| Y<br>A | JP 61-126020 A (Sekisui Chemical Co., Ltd.,<br>Meiji Seika Kaisha, Ltd.),<br>13 June 1986 (13.06.1986),<br>claim 1<br>(Family: none) | 14<br>1-13,15 |
| Y<br>A | JP 62-181226 A (Ikeda Mohando Ltd.),<br>08 August 1987 (08.08.1987),<br>claim 2<br>(Family: none) | 14<br>1-13,15 |
| Y<br>A | JP 7-316075 A (Pola Chemical Industries Inc.),<br>05 December 1995 (05.12.1995),<br>claim 7<br>(Family: none) | 14<br>1-13,15 |

Form PCT/ISA/210 (continuation of second sheet) (July 2009)

**INTERNATIONAL SEARCH REPORT**

International application No.

PCT/JP2010/001761

Continuation of A. CLASSIFICATION OF SUBJECT MATTER
  (International Patent Classification (IPC))

A61K47/34(2006.01)i, A61P29/00(2006.01)i

          (According to International Patent Classification (IPC) or to both national
          classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 61126020 A
- JP 62126119 A
- JP 58039616 A
- JP 58103311 A
- JP 6009394 A
- JP 2002020274 A
- JP 3251534 A
- JP 1143831 A
- JP 2000143540 A

### Non-patent literature cited in the description

- Drugs in Japan, Ethical drugs, 2006. Jiho, Inc, 2006, 221